Europäisches Patentamt

**European Patent Office**

**Office européen des brevets**

(11) Publication number: **0 045 137**
**A2**

(12) # EUROPEAN PATENT APPLICATION

(21) Application number: **81302922.0**

(22) Date of filing: **29.06.81**

(51) Int. Cl.³: **A 61 B 19/08**, A 61 F 13/00

(30) Priority: **15.07.80 GB 8023036**
**20.09.80 GB 8030454**

(43) Date of publication of application: **03.02.82**
**Bulletin 82/5**

(84) Designated Contracting States: **AT BE CH DE FR IT LI NL SE**

(71) Applicant: **Smith and Nephew Associated Companies Limited, 2, Temple Place Victoria Embankment, London WC2R 3BP (GB)**

(72) Inventor: **Lloyd, Ronald, 63 Cambridge Road, Sawbridgeworth Hertfordshire (GB)**
Inventor: **Berry, Peter William, 20 Marlborough Close, Bishops Stortford Hertfordshire (GB)**

(74) Representative: **Cole, William Gwyn, Corporate Patents Department Smith and Nephew Research Limited Gilston Park, Harlow Essex CM20 2RQ (GB)**

(54) **Surgical drape fabric.**

(57) A moisture vapour transmitting, water impermeable drape fabric which comprises an absorbent layer and a porous layer bonded together by a pressure sensitive adhesive which prevents the passage of bacteria between the two layers and methods for the production thereof and sterile surgical drapes and gowns made therefrom are described.

-1-

## SURGICAL DRAPE FABRIC AND DRAPES

The present invention relates to absorbent surgical drapes and methods of their manufacture and to the fabric therefor.

Absorbent surgical drapes are used inter alia around operation sites where their function is to form a barrier between the site and the surrounding skin areas to aid in preventing infection. The upper layer of the drape should be capable of absorbing fluids such as blood and the lower layer of the drape should be capable of transmitting fluids such as perspiration. The central layer of the drape prevents communication of the two layers.

It is known from British Patent Specification No. 1061054 to form surgical drapes with two absorbent layers laminated to a central layer of a polyethylene film. Unfortunately drapes using a polyethylene barrier

-2-

prevent evaporation of perspiration and can cause a
clammy feeling.

It is known from British Patent Specification No.
1341605 to form surgical drapes and gowns in which
two absorbent layers are bonded to a central layer of a
water vapour permeable film or sheet substrate which may
be made of non-adhesive material.

A surgical drape fabric has now been discovered
that has good moisture vapour transmission rates while
avoiding the use of an independent central film or sheet
which could reduce the flexibility of the fabric.

The present invention provides a moisture vapour
transmitting, water impermeable drape fabric which
comprises an absorbent layer, and a porous layer bonded
together by a pressure sensitive adhesive which prevents
the passage of bacteria between the two layers.

When used herein the term 'porous layer' means
a layer having pores or openings which allow the
transmission of moisture vapour through the layer.

The moisture vapour transmission rate of the drape
of this invention is suitably not less than $200g/m^2$, more

suitably not less than $250g/m^2$ and preferably bot less than $500g/m^2/24hrs$ at $37^{o}C$ at 100%-10% relative humidity difference.

The moisture vapour transmission rate may be measured by the Payne Cup method. The method uses a cup 1.5cm deep with a flanged top. The inner diameter of the flange is such as to provide an area for moisture vapour transmission of $10cm^2$. In this method 10ml. of distilled water is added to the cup and a sample of the material under test, large enough to completely cover the flange, is clamped over the cup. The complete assembly is then weighed and placed in a cabinet where the temperature and relative humidity are maintained at $37^{o}C$ and 10% respectively. After 17 hours, the cup is removed from the cabinet and allowed to cool at room temperature. After re-weighing, the mass of water lost by vapour transmission is calculated and the result expressed as $g/m^2/24hr$. at $37^{o}C$, 100%-10% relative humidity difference.

In use the two external layers of surgical drape materials can perform different functions. The outer layer should be capable of absorbing water and aqueous body fluids such as blood. The rate of absorption of this layer should be rapid enough to prevent liquids running off the surface of the surgical drape fabric and thus contaminating other surfaces. It is advantageous that the outer absorbent layer should also have the ability to absorb other liquids such as isopropyl alcohol used at or around the operation site during certain surgical procedures. It is also desirable that the outer absorbent layer should not shed particles or fibres and should have a "non-pilling" surface in the presence of moisture.

The inner layer which in use is adjacent to the patients skin should be capable of transmitting perspiration in either liquid or vapour form, produced during the period of the operation, thereby reducing the "clammy" feeling caused by the build-up of moisture and heat under the drape.

Suitable porous layers include non-woven fabrics, foams and porous or microporous films. The porous layer can be absorbent or non absorbent. Normally and preferably it is absorbent.

In a favoured aspect the present invention provides a moisture vapour transmittingm water impermeable drape fabric which comprises of two absorbent layers bonded together by a pressure sensitive adhesive which prevents the passage of bacteria between the absorbent layers.

It is preferred that in this form of the invention the inner absorbent layer should have similar properties to that of the outer layer, although the absorbent capacity of the inner layer may be lower if desired.

In one preferred construction of the surgical drape fabric the two absorbent layers are made of the same material.

In another preferred construction the absorbent layers are different. The two absorbent layers may have different chemical composition, physical construction or different weights or thicknesses.

Suitable materials for the absorbent layers include non-woven fabrics, foams and treated papers.

Especially suitable materials for the absorbent layers include bonded non-woven fabrics containing viscose rayon fibres, their blends with wood pulp fibres and treated 'spun-bonded' polyester fibre non-woven fabrics. An apt weight range for the non woven fabric for the outer absorbent layer is 20-70gsm and preferred weight range is 25-45gsm. Similar weight of material may be used for the inner absorbent layer but if desired a lower weight such as 15-20 gsm may be employed.

A suitable polyester 'spun-bonded' staple fibre non-woven fabric which is heat set and has been given an "anti-pill" finish is available as 'Sontara' (trade mark) from Dupont. Sontara 3008 which has a base weight of approximately 70 gsm/sq. metre and Sontara 8000 which has a base weight of approximately 40gsm are both apt.

A suitable apertured non-woven fabric made from spun-bonded viscose rayon filaments is available as Bemliese (Trade Mark) from Asahai Chemical Industry Co. Ltd. Bemliese is available in weights ranging from 18-45gsm which are suitable for use in this invention.

A suitable bonded non-woven fabric made from viscose rayon staple fibre is available as Paraprint (Trade Mark) from Lohmann GmbH & Co. KG. Grade OL 35 which has a base weight of 35gsm and grade OL 20 which has a base weight of 20 gsm are both apt.

A suitable bonded non-woven fabric made from a blend of wood pulp fibres and viscose rayon fibres is available as Storalene from Stora Kopparsberg Bervik Ltd. Grades 509.17 and 715.45 have base weights of 18 gsm and 43 gsm respectively are both apt.

A suitable bonded non-woven fabric made from a blend of viscose rayon and wood pulp fibres is available, Tamlon (Trade Mark) from Tampella. Grade K 267/40 has a base weight of 44gsm and is apt for use in this invention.

Suitable non-absorbent porous layers include non-woven fibrous materials made by stretching melt embossed polymer blends. One form of preferred non-woven fibrous reticulate material is disclosed in British Patent Specification No. 1531715. A second form of preferred non-woven fibrous reticulate material is disclosed in British Patent Specification No. 1548865. A further form of a non absorbent porous layer is disclosed in British Patent Specification No. 1495151.

The pressure sensitive adhesive can be in any form providing it prevents the passage of bacteria between the two layers and has a moisture vapour transmission rate of at least $200g/m^2/24hrs$. The pressure sensitive adhesive will also act as a barrier to liquid water and aqueous body fluids such as blood. It is desirable that the pressure sensitive adhesive has some resistance to the penetration of isopropyl alcohol and preferable that it prevents the penetration of isopropyl alcohol.

Normally and preferably the pressure sensitive adhesive is continuous (as opposed to microporous; naturally the adhesive will always be macroscopically continuous).

-8-

Most aptly the adhesive has a moisture vapour transmission rate of at least $250g/m^2$ and preferably at least 500 $g/m^2/24hrs./at 37^{O}C.$ at 100% - 10% relative humidity difference.

Suitable pressure sensitive adhesives which are moisture vapour permeable as a continuous coating include acrylate ester copolymers, polyvinyl ethyl ethers and polyurethanes. Examples of suitable adhesives are given in British Patent Specification No. 1280631.

The pressure sensitive adhesives can be polymers per se, blends of polymers, mixtures of polymers with tackifying resins and optionally other materials such as fillers and antioxidants.

A preferred pressure sensitive adhesive comprises a blend of high and low viscosity polyvinyl ethyl ethers in particular "adhesive composition A" disclosed in British Patent Specification No. 1280631.

Other preferred pressure sensitive adhesives comprise copolymers of acrylate ester with acrylic acid and in particular a copolymer of 47 parts by weight of n-butyl acrylate, 47 parts by weight of ethylhexyl acrylate and 6 parts by weight of acrylic acid polymerised in acetone according to the general method given in U.S. Specification No. 2884126 and with an intrinsic viscosity of at least 1.8 dl/g.

Another preferred pressure sensitive adhesive is a crosslinkable acrylate ester copolymer available as a 55% solids aqueous emulsion and known as Primal N 580 (Trade Mark) made by Rohm and Haas. Primal N 580 is preferably used in a thickened form. An acrylic thickener known as Primal AS E60 (Trade Mark) made by Rohm and Haas is particularly suitable for thickening Primal N 580. A coating of Primal N 580 when dried gives a crosslinked pressure sensitive adhesive.

Crosslinked pressure sensitive adhesives will have a better resistance to solvents than the uncrosslinked adhesives and therefore drape fabrics·bonded with a crosslinked pressure sensitive adhesive are a preferred aspect of the invention.

The weight range of the adhesive coating is generally from 15 to 90 gsm, desirably 15 to 70 gsm and preferably from 20 to 45 gsm.

The adhesive coating can be formed by any convenient method including solution and emulsion coating, coating from a hot melt, by extrusion and also spray coating.

The present invention also provides a method of forming a moisture vapour transmitting surgical drape fabric as hereinbefore defined which comprises bonding together an absorbent layer and a porous layer with a pressure sensitive adhesive.

A favoured method of production of the drape fabric is to form the adhesive on the surface of at least one layer by transfer coating process and to bond the other layer thereto by lamination under pressure.

To avoid random pinholes in the adhesive which may occur when the adhesive is formed as a single coating on for example a release surface it is preferred that the continuous adhesive comprises two or more coatings.

A favoured method is to form separate adhesive coatings on both non woven fabric layers for example by transfer coating and to bond the layers together at the adhesive surfaces under light pressure. In an alternative method the pressure sensitive adhesive can be formed by applying two or more coatings for example by spraying to a non woven fabric layer. Combinations of both these methods can also be used.

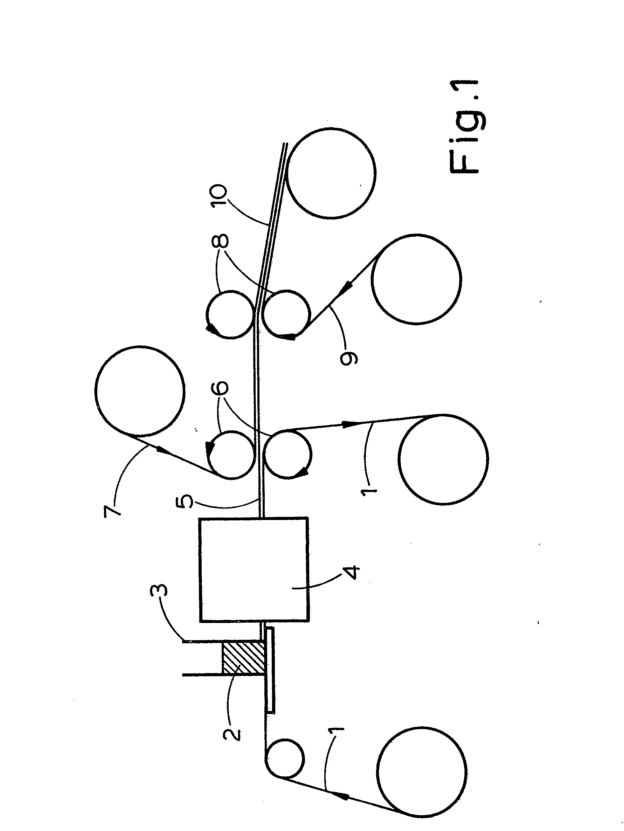

Figure 1 illustrates a process of making the

-11-

surgical drape fabric of the invention. A silicone release paper (1) is coated with a solution or emulsion of the pressure sensitive adhesive (2) by means of a coating head (3) and passed through a drying oven (4). The dried continuous coating (5) of pressure sensitive adhesive on the release paper (1) is passed through the nip of pressure rollers (6) where it is transferred to a non-woven fabric (7) and stripped from the release paper. The adhesive coated non-woven fabric is then passed through the nip of another set of pressure rollers (8) where it is laminated to a second non-woven fabric (9) to form the surgical drape fabric (10) of the invention.

The surgical drape fabric can be processed in the various forms and sizes and packaged.

Suitable forms of surgical drape include square or rectangular sheets with or without fenestration. Useful sizes are 71cm x 91cm (small) 91cm x 91cm, 91cm x 142cm (medium) 142cm x 142cm (large) and 142cm x 178cm (extra large).

The surgical drape can be made up in a suitable form for particular surgical procedures for example in the form of leggings for lithotomy.

-12-

The surgical drape can be provided with a pressure sensitive adhesive margin to enable the drape to be adhered to the patient. The adhesive margin can be located at one or more outside edges of the drape or around the inner edge or edges of a fenestrated area of the drape.

When the surgical drape has a square or rectangular shape it is desirable that the surgical drape has a pressure sensitive adhesive margin on one edge or on two opposed edges. It is also desirable that a fenestrated surgical drape should have a continuous pressure sensitive adhesive margin around the edge or edges of the fenestration.

The pressure sensitive adhesive margin can be formed by coating or laminating an additional pressure sensitive adhesive layer onto the margins of the drape. Alternatively the pressure sensitive adhesive margin can be provided by exposing the surface of the pressure sensitive adhesive used to bond the layers together. In one form this is acheived by bonding a first layer coated with the pressure sensitive adhesive to a second layer of lesser area than that of the first layer. For example surgical drapes of rectangular or square shape with a pressure sensitive adhesive margin on one edge

-13-

or opposed edges can be made from a surgical drape fabric which is formed by bonding a first layer of a non-woven fabric coated with a moisture vapour transmitting pressure sensitive adhesive to a second narrower layer of non-woven fabric.

In a second and preferred form this is acheived by removing a strip or strips of a tearable porous film used to form the porous layer of surgical drape of the invention. Suitable tearable porous films include those described in British Patent Specification No. 1495151. A preferred porous film is described in Example 1 of the above specification.

The width of the pressure sensitive adhesive margin can depend on the size of the surgical drape but a width of about 2.5cm to7.5cm is desirable. It is desirable that the pressure sensitive adhesive margins of the surgical drape should be covered by a removable protector.

The surgical drapes can be individually packaged in sealed paper bags or film/paper pouches which are bacteria proof under dry conditions. Alternatively the drape can be part of a procedure pack.

-14-

It is preferable that the surgical drape is sterilised in its packaged condition. Any convenient means of sterilisation may be employed such as steam autoclaving, gas sterilisation techniques and irradiation with elecron or gamma rays.

The use of a pressure sensitive adhesive to bond the absorbent layers together has advantages that the surgical drape fabrics of this invention can be manufactured by a simple pressure laminating process and the resultant drape fabrics can be flexible and less "boardy" than drape materials made with a preformed central film layer.

The surgical drape fabric of this invention is also suitable for forming surgical gowns and other aseptic absorbent barrier materials which are used in conjuction with the skin.

Gowns made from the surgical drape form an aspect of this invention.

Example 1

A polyvinyl ethyl ether based pressure sensitive adhesive composition ("Adhesive composition A" of British Patent specification No. 1280631) was coated using a knife over flat bed coating unit onto a silicone release coated paper (Steralease 77) and dried in an oven at 80$^O$C to give a dry coating weight of 29 gsm. The pressure sensitive adhesive coated release paper was then laminated to Tamlon grade 267/40 non-woven fabric by passage through the nip of two rollers under pressure and the release paper stripped off. The adhesive coated non-woven fabric was then laminated to Storalene grade 509/17 non-woven fabric by passage through the nip of another set of rollers under pressure.

The surgical drape fabric of the Example had a moisture vapour transmission rate of 2000g/m$^2$/24hrs at 37$^O$C at 100%-10% relative humidity difference.

The fabric had a weight of 90gsm and a thickness of 0.28mm. The drape fabric rapidly absorbed water placed on either surface which did not penetrate to the other surface in either case.

Example 2

In a similar manner to Example 1 a surgical drape fabric was made using a pressure sensitive adhesive composition consisting of a copolymer of 47 parts by weight of ethyl hexyl acrylate, 47 parts by weight of n-butyl acrylate, 6 parts by weight of acrylic acid polymerised in acetone according to the general method of U.S. Specification No. 2884126 with an intrinsic viscosity of 1.9 dl/g. A dry coating weight of 64 gsm was used.

The surgical drape fabric of the example had a moisture vapour transmission rate of $900g/m^2/24$ hrs at $37^{\circ}C$ at 100%-10% relative humidity difference. The drape fabric had a weight of 125 gsm and thickness 0.35mm.

The drape fabric rapidly absorbed water placed on either surface which did not penetrate to the other surface.

Example 3

In a similar manner to Example 1 a surgical drape fabric was made using 30gsm of the acrylate ester copolymer adhesive of Example 2 to bond together a Paraprint grade OL 35 absorbent non-woven fabric to a non-woven fibrous reticulate material as described in Example 1 of British Patent Specification No. 1548365.

The surgical drape fabric of the example had a moisture vapour transmission rate of about 1600 $g/m^2/$ 24 hrs at 37$^O$C at 100% - 10% relative humidity difference. The drape fabric had a weight of 77 gsm and a thickness of 0.25mm.

The drape fabric rapidly absorbed water placed on the absorbent non-woven fabric surface which did not penetrate to the outer surface.

Example 4

An acrylate ester copolymer aqueous emulsion adhesive (Primal N 580 supplied by Rohm and Haas (U.K.) Ltd.) thickened by the addition of an acrylic thickener (24 parts by weight per hundred parts of the adhesive emulsion of 10% aqueous solution of Primal ASE 60 supplied by Rohm and Haas (U.K.) Ltd.) and maintained at a pH 9 by the addition of ammonium hydroxide was coated onto a silicone release polyethylene coated paper (Steralease 15 supplied by Sterling Coated Papers Ltd.) by a knife over flat bed coating unit and dried in an oven at 95$^O$C for 2 minutes to give a dry coating weight of 30g/m$^2$. The Pressure sensitive adhesive coated release paper was then laminated to Storalene grade 509.17 non woven fabric by passage through the nip of two rollers, heated to approximately 50$^O$C under light pressure. In a similar manner another length of the pressure sensitive adhesive coated paper was laminated to Paraprint grade OL 25 non-woven fabric. The release paper was then removed from both non-woven fabric laminates and the adhesive coated non-woven fabrics bonded together at their adhesive surfaces by passage through the nip of two rollers heated to 55°C under slight pressure.

The surgical drape fabric of the example had a moisture vapour transmission rate of approximately 1550g/$m^2$/24 hours at 37°C at 100% - 10% relative humidity difference.

Example 5

In a similar manner to Example 4 a surgical drape fabric was made using the pressure sensitive adhesive of Example 2 instead of emulsion adhesive Primal N 580. The surgical drape fabric of the example had a moisture vapour transmission rate of approximately 900g/$m^2$/24 hours at 37°C at 100% to 10% relative humidity difference.

Example 6

In a similar manner to Example 4 a surgical drape fabric was made using a polyvinyl ethyl ether pressure sensitive adhesive consisting of 32.3 parts by weight of polyvinyl ethyl ether K value 100 (Lutonal A 100, a 25% solution in hexane available from B.A.S.F.), 53.3 parts by weight of polyvinyl ethyl ether K value 50 (Lutonal A 50 a 70% solution in petroleum solvent available from B.A.S.F.), 13.7 parts by weight of a zinc resinate (Kelrez 42-263, a polymerised gum derivative containing 7.9% to 8.5% zinc, available from Croda Resins Ltd.) and 0.7 parts by weight of an antioxidant (Permanax W.S.L. available from Vulnax Limited) dissolved in a petroleum ether solvent (SBP2, boiling point range $72^{O}C - 95^{O}C$, available from BP Chemicals Limited) to give 40% solids.

The surgical drape fabric of the example had a moisture vapour transmission rate of approximately 1150 g/m$^2$/24 hours at $37^{O}C$ at 100% - 10% relative humidity difference.

Isopropyl Alcohol Penetration Test

The resistance of a drape fabric to penetration by isopropyl alcohol was tested by placing a small drop of isopropyl alcohol at five locations on one side of a 10cm x 10cm square sample of the drape fabric. After a period of 1 minute the reverse side was examined for penetration of the liuqid. Drape fabric samples from Examples 4, 5 and 6 were subjected to this test. No penetration of the isopropyl alcohol was observed.

Bacteria Penetration Test.

The resistance of a drape fabric to penetration by bacteria was tested by placing a 10cm x 10cm test sample of the drape fabric onto the surface of an agar plate so that the sample overlapped the edge of the plate. 0.2ml. of a 24 hour broth culture of *serratia marcescens* (motile red pigmented organism approximately 0.5 microns in diameter) was spread over the top surface portion of the test sample in contact with the agar plate so as to leave the edge margins of the sample uncoated and the test sample left at room temperature for 4 hours. The drape sample was then removed and the agar plate incubated at $28^{O}$ to $3o^{O}C$ for 24 hours. Any penetration of the test organism through the drape sample is indicated by growth of the pigmented organism on the agar plate.

Drape fabric samples of Examples 4, 5 and 6 were found to have good resistance to penetration by the test organism.

Drape fabric samples of Example 4 and 5, sterilised by autoclaving at $134^{O}C$ for 3.5 minutes, also showed good resistance to penetration by the test organism.

WHAT WE CLAIM IS:-

1.      A moisture vapour transmitting water impermeable drape fabric which comprises an absorbent layer and a porous layer bonded together characterised in that the absorbent layer and porous layer are bonded together by a pressure sensitive adhesive which prevents the passage of bacteria between the two layers.

2.      A drape fabric as claimed in claim 1 having a moisture vapour transmission of not less than $250g/m^2/$ 24 hours at $37^{O}C$ at 100% - 10% relative humidity difference.

3.      A drape fabric as claimed in either of claims 1 or 2 in which the pressure sensitive adhesive is continuous and has a weight of $15g/m^2$ to $70g/m^2$.

4.      A drape fabric as claimed in claim 3 in which the pressure sensitive adhesive comprises an optionally crosslinked alkyl acrylate copolymer or polyvinyl ethyl ether.

5.      A drape fabric as claimed in any of claims 1 to 4 in which the porous layer comprises a non woven fibrous reticulated material.

6.      A drape fabric as claimed in any of claims 1 to 4 in which the absorbent layer and the porous layer comprise an absorbent bonded non woven fabric.

7.      A drape fabric as claimed in claim 6 in which the bonded non woven fabric of the outer absorbent layer has a weight of $25g/m^2$ to $4.5 \ g/m^2$ and the bonded non woven fabric of the inner absorbent layer intended to be used adjacent to the patient's skin has a weight of $15g/m^2$ to $20g/m^2$.

8.      A method for forming a moisture vapour transmitting surgical drape fabric as defined in any of claims 1 to 7 which comprises bonding together an absorbent layer and a porous layer with a pressure sensitive adhesive.

9.      A sterile surgical drape or gown made from a drape fabric as defined in any of claims 1 to 7.

10.     A sterile surgical drape as claimed in claim 9 in which a pressure sensitive adhesive margin is provided at one or more edges of the drape, said margin being provided by exposing the surface of the pressure sensitive adhesive used to bond the layers together.

Fig.1